Europäisches Patentamt

European Patent Office

Office européen des brevets

(11) Veröffentlichungsnummer : **0 092 200 B1**

# EUROPÄISCHE PATENTSCHRIFT

(45) Veröffentlichungstag der Patentschrift :
30.11.88

(21) Anmeldenummer : 83103672.8

(22) Anmeldetag : 15.04.83

(51) Int. Cl.⁴ : **A 61 L 15/04**

(54) **Resorbierbares Flachmaterial zum Abdichten und Heilen von Wunden und Verfahren zu dessen Herstellung.**

(30) Priorität : 19.04.82 DE 3214337

(43) Veröffentlichungstag der Anmeldung :
26.10.83 Patentblatt 83/43

(45) Bekanntmachung des Hinweises auf die Patenter-teilung : 30.11.88 Patentblatt 88/48

(84) Benannte Vertragsstaaten :
AT BE CH DE FR GB IT LI LU NL SE

(56) Entgegenhaltungen :
EP-A- 0 049 469
EP-A- 0 059 265
EP-A- 0 068 047
EP-A- 0 068 048
EP-A- 0 068 149
FR-A- 2 448 900
BIOMATERIALS, Band 3, Nr. 2, 1982, Seiten 669-676, John Wiley and Sons Ltd.; H. REDL et al.: "Back-ground and methods of "fibrin sealing""

(73) Patentinhaber : Serapharm GmbH & Co. KG
Bohlweg 20
D-4400 Münster (DE)

(72) Erfinder : Zimmerman, Eberhard, Prof. Dr.
Am Braaken 16
D-4400 Münster-Nienberge (DE)
Erfinder : Stroetmann, Michael
Kaiser-Wilhelm-Ring 36
-4400 Münster (DE)

(74) Vertreter : Brehm, Hans-Peter, Dr. Dipl.-Chem. et al
Patentanwälte Kern, Brehm & Partner Albert-Ross-haupter-Strasse 73
D-8000 München 70 (DE)

## Beschreibung

Die Erfindung betrifft ein resorbierbares Flachmaterial zum Abdichten und Heilen von Wunden, das im wesentlichen aus einer Glykoprotein-Matrix besteht, mit einem Gehalt an die Blutgerinnung bewirkenden bzw. fördernden Substanzen einschl. Fibrinogen und Thrombin.

Es ist bereits ein Flachmaterial zum Heilen von Wunden bekannt, dessen Matrix aus Gelatine, Collagen, Polyglykolsäure oder Polymilchsäure bestehen kann, an welchem Matrix-Material der Blutcoagulationsfaktor XIII und Thrombin fixiert ist (vgl. DE-A-29 14 822). Dieses bekannte Flachmaterial enthält kein Fibrinogen.

Weiterhin ist ein als « Fibrinkleber » bezeichneter Gewebeklebstoff bekanntgeworden (vgl. DE-A-30 02 933), der hauptsächlich aus einer tiefgefrorenen Fibrinogen-Lösung mit Zusätzen von Faktor XIII und einem Fibrinolyse-Inhibitor wie etwa Aprotenin besteht. Zur praktischen Anwendung taut man die tiefgefrorene Fibrinogen-Lösung auf, versetzt mit einer Lösung von Thrombin und Calciumchlorid, hält das Gemisch eine zeitlang, bis sich die einsetzende Polymerisationsreaktion durch eine Viskositätssteigerung bemerkbar macht und bringt dann dieses reagierende Gemisch auf den zu verbindenden Gewebeteilen auf. Der Aufwand zur Bereitung des einsatzfähigen Gewebeklebers und die geringe Lebensdauer des einsatzbereiten Präparates hat sich in vielen Fällen als hinderlich erwiesen.

Weiterhin ist auch schon vorgeschlagen worden (Wiener Medizinische Wochenschrift 7, S. 86 bis 89, 1976), lyophilisiertes Fibrinogen auf 37 °C zu erwärmen, die erhaltene Lösung auf ein Kollagenvlies aufzubringen und dort durch den Zusatz einer wässerigen Lösung von Thrombin und einer wässerigen Lösung von Faktor XIII zur Gerinnung zu bringen. Das reaktive Flachmaterial wird daraufhin auf die Wunde übertragen. Auch in diesem Falle bereitet die Handhabung Schwierigkeiten, da das einsatzbereite Flachmaterial unmittelbar vorher hergestellt werden muß, und das kurzzeitige Intervall des einsatzbereiten Präparates nicht sicher erfaßt werden kann.

Da Thrombin in wässerigem Medium ja bereits in feuchter Umgebung die Polymerisation und Vernetzung von Fibrinogen zu Fibrin bewirkt, weisen Präparate, die sowohl Thrombin wie Fibrinogen enthalten, zumeist eine schlechte Lagerfähigkeit auf, sofern nicht besondere Maßnahmen zum vollständigen oder weitgehenden Wasserausschluß getroffen werden.

Das nicht vorveröffentlichte Dokument EP-A-59 265 (Stand der Technik nach Art. 54 Abs. 3 EPÜ) offenbart ein Material zum Abdichten und Heilen von Wunden, bestehend aus einem Kollagenträger, einer Fibrinogenkomponente und einer Thrombin-Komponente. Die Fibrinogen- und die Thrombinkomponente und gegebenenfalls zusätzliche Wahlkomponenten, wie Calciumionen, Proteaseninhibitoren, Heparinantagonisten, die Einsprossung und das Wachstum von Fibroplasten fördernde Substanzen, wie Fibronectin, sowie infektionshemmende Arzneimittel sind in Form einer Beschichtung vorhanden, die einseitig oder allseitig auf dem Kollagenträger aufgetragen ist. Typischerweise bilden die Fibrinogen- und die Thrombinkomponente eine Feststoffmischung, deren Partikel an der Oberfläche des Kollagenträgers haften. Wichtig ist das Nebeneinander-Vorhandensein von Fibrinogenpartikeln und Thrombin- bzw. thrombinfreisetzenden Partikeln auf einem Kollagenträger, ohne daß sie miteinander reagieren. Eine vorzeitige, unbeabsichtigte Fibrinbildung soll dadurch vermieden werden, daß bei der Aufbringung der Komponenten auf dem Kollagenträger die Anwesenheit von erheblichen Mengen Wasser verhindert wird. Vielmehr kann eine feinteilige Mischung der Komponenten in einem organischen, weitgehend wasserfreien Lösungsmittel suspendiert und diese Suspension auf dem Träger aufgesprüht werden. Die praktische Erfahrung zeigt jedoch, daß unter diesen Bedingungen eine teilweise Denaturierung der Gerinnungsenzyme auftreten kann, die zu einem Aktivitätsverlust der Enzyme führen kann.

Das nicht vorveröffentlichte Dokument EP-A-68 047 (Stand der Technik nach Art. 54 Abs. 3 EPÜ) offenbart ein angereichertes Plasmaderivat zur Unterstützung von Wundverschluß und Wundheilung. Im wesentlichen handelt es sich um ein Pulvergemisch, das vorzugsweise 60 bis 96 Gew.-% Fibrinogen, 0,1 bis 15 Gew.-% Fibrinolyse-Inhibitor enthalten kann ; weiterhin können zusätzlich Wahlkomponenten wie Phospholipide, Prostaglandine, Trockenhalte- und Stabilisierungsmittel (Albumin, Globuline), Antibiotika, Blutgerinnungsfaktoren, Blutplättchenextrakte vorhanden sein.

Dieses Plasmaderivat ist zur unmittelbaren Applizierung auf einer Wunde bzw. im Operationsgebiet bestimmt und hierfür als trockenes Pulvergemisch oder zusammen mit einem die Bestandteile nicht lösenden Treibmittel als Spray oder Schaum konfektioniert. In jedem Falle ist eine enge und unmittelbare räumliche Nachbarschaft von Fibrinogen und Thrombin bzw. Prothrombin gegeben.

Das nicht vorveröffentlichte Dokument EP-A-68 048 (Stand der Technik nach Art. 54 Abs. 3 EPÜ) offenbart ein weiteres angereichertes Plasmaderivat zur akzelerierten Haemostase und optimierten Regelung des Wundverschlusses, zu dessen Hauptbestandteilen Thrombin, das teilweise durch Komponenten des Prothrombinkomplexes ersetzt sein kann, Fibrinolyse-Inhibitoren und wenigstens ein Trockenhalte- und Stabilisierungsmittel aus der Albumin, Globulin und Fibrinogen umfassenden Gruppe gehören, und das gegebenenfalls zusätzliche Beimischungen von Blutplättchenextrakten, Antibiotika und dergleichen enthalten kann, dessen Bestandteile ausnahmslos in pulverförmigem Zustand vorliegen, und das als Spray konfektioniert ist. Bei einer beispielhaften Zusammensetzung besteht das Pulvergemisch aus 20 bis 50 Gew.-% Thrombin, 5 bis 40 Gew.-% Prothrombin, 8 bis 70 Gew.-% Albumin, Globulin und/oder Fibrinogen, 3 bis 12 Gew.-% wasserlöslichem Kollagen, 0,5 bis 1,2 Gew.-% Plättchenextrakt und 2 bis 6 Gew.-% Fibrinolyse-Inhibitor. Sofern das Pulvergemisch Thrombin und Fibrinogen enthält, ist eine enge und unmittelbare räumliche Nachbarschaft der Fibrinogen- und Thrombin-Partikeln gegeben.

Das nicht vorveröffentlichte Dokument EP-A-68 149 (Stand der Technik nach Art. 54 Abs. 3 EPÜ) offenbart ein fibrinogenhaltiges Trockenpräparat mit einer durch Gefriertrocknung erzielten Schaum- bzw. Vliesstruktur, das neben Thrombin in zumindest katalytisch wirksamen Mengen im wesentlichen aus etwa 10 bis 95 Gew.-% Fibrin und etwa 5 bis 90 Gew.-% Fibrinogen besteht. Zur Herstellung wird in einer Fibrinogen und Thrombin enthaltenden wässerigen Lösung in situ Fibrin erzeugt, und das entstehende Reaktionsgemisch tiefgefroren und lyophilisiert. Als weitere Bestandteile des Trockenpräparates kommen Wirkstoffe, wie etwa Antibiotika natürliches Knochenmaterial und/oder synthetischer, knochenbildender Ersatzstoff, Glykoproteine, gerinnungsfördernde Substanzen und dergleichen, und/oder Fibrinolyse-Inhibitoren in Betracht. Das Trockenpräparat ist vor allem als Wundversorgungsmaterial, Füllmaterial für Knochenhohlräume und/oder als Trägermaterial für weitere Wirkstoffe vorgesehen. Aufgrund der Herstellungsweise ist das in geringen Mengen vorhandene Thrombin homogen innerhalb des gesamten Trockenpräparates verteilt.

Die Aufgabe der vorliegenden Erfindung besteht darin, ein Flachmaterial zum Abdichten und Heilen von Wunden bereitzustellen, das Thrombin und Fibrinogen enthält, das bei Umgebungstemperaturen ohne nennenswerten Aktivitätsverlust über längere Zeiträume lagerbar ist, und das unmittelbar, d. h. ohne die Zugabe weiterer, beispielsweise aktivierender Komponenten auf der Wunde appliziert wird und dort vollständig resorbierbar ist. Nach einem weiteren Ziel der Erfindung soll das Flachmaterial zusätzlich ein oder mehrere, speziell die Wundheilung unterstützende Mittel enthalten.

Ferner soll mit dieser Erfindung wenigstens ein Verfahren zur Herstellung eines solchen Flachmaterials angegeben werden, das einfach durchführbar ist, die biologische Aktivität von Fibrinogen und Thrombin und gegebenenfalls der(des) speziell die Wundheilung unterstützenden Mittel(s) im Laufe der Herstellung nicht beeinträchtigt und das eine große Variationsbereite hinsichtlich der Art, Anzahl, Konzentration und Anordnung der Komponenten innerhalb des Flachmaterials erlaubt.

Ausgehend von einem resorbierbaren Flachmaterial zum Abdichten und Heilen von Wunden, das im wesentlichen aus einer Glykoprotein-Matrix besteht und einen Gehalt an die Blutgerinnung bewirkenden bzw. fördernden Substanzen einschließlich Fibrinogen und Thrombin aufweist, ist die erfindungsgemäße Lösung obiger Aufgabe dadurch gekennzeichnet, daß das Flachmaterial trocken und mehrschichtig ausgebildet ist, wenigstens eine Teilschicht des mehrschichtigen Flachmaterials Thrombin-frei ist und das Fibrinogen in deren Glykoprotein-Matrix weitgehend homogen verteilt enthält ; und wenigstens eine weitere Teilschicht des mehrschichtigen Flachmaterials Fibrinogen-frei ist und das Thrombin in deren Glykoprotein-Matrix weitgehend homogen verteilt enthält.

Nach einem weiteren bevorzugten Gesichtspunkt der Erfindung enthält wenigstens eine Teilschicht — vorzugsweise wenigstens eine der Außenschichten — des Flachmaterials zusätzlich in trockenem Zustand vorliegende Fibroplasten-Zellen.

Nach einem weiteren bevorzugten Gesichtspunkt der Erfindung enthält wenigstens eine Teilschicht — vorzugsweise wenigstens eine der Thrombin und Fibroplasten-Zellen enthaltende Außenschichten — des Flachmaterials zusätzlich ein oder mehrere das Wachstum und die Einsprossung von Fibroplasten-Zellen fördernde(s) Mittel.

Das erfindungsgemäße Verfahren zur Herstellung eines solchen Flachmaterials sieht vor, eine Thrombin-freie, überwiegend wässrige Lösung und/oder Suspension des Glykoproteins mit Fibrinogen zu versetzen und zu einer Flachmaterial-Teilschicht zu verarbeiten, eine weitere, überwiegend wässrige, Fibrinogen-freie Lösung und/oder Suspension des nämlichen oder eines anderen Glykoproteïns mit Thrombin zu versetzen und zu einer weiteren Flachmaterial-Teilschicht zu verarbeiten, und diese Teilschichten, gegebenenfalls mit weiteren, auf ähnlichem Wege erzeugten Teilschichten vollflächig aufeinander aufzubringen. Vorzugsweise wird die Thrombin-haltige Lösung und/oder Suspension des Glykoproteins mit aktiven Fibroplasten-Zellen und/oder mit einem das Wachstum sowie die Einsprossung von Fibroplasten förderndem Mittel versetzt. Vorzugsweise dient als das Wachstum und die Einsprossung von Fibroplasten förderndes Mittel Chondroitinsulfat.

Eine besonders bevorzugte Ausführungsform dieses Verfahrens sieht vor, eine Lösung und/oder Suspension einer Zusammensetzung der genannten Art auf einer inerten Oberfläche aufzubringen und dort tiefzufrieren, und auf der erhaltenen Eisschicht-Oberfläche noch im tiefgefrorenen Zustand eine weitere Lösung und/oder Suspension anderer Zusammensetzung ebenfalls tiefzufrieren und schließlich sämtliche Teilschichten gemeinsam zu lyophilisieren. Gegebenenfalls kann die aufgebrachte Lösung und/oder Suspension vor dem Tieffriervorgang verschäumt werden.

Vorteilhafte Ausgestaltungen und Weiterbildungen der Erfindung ergeben sich aus den Unteransprüchen.

Die Erfindung schafft ein vollständig resorbierbares Flachmaterial zum Abdichten und Heilen von Wunden, das unter sterilen, trockenen Bedingungen bei Raumtemperatur über längere Zeiträume, beispielsweise 2 Jahre und mehr ohne nennenswerten Aktivitätsverlust lagerbar ist und das nach der Entnahme aus seiner sterilen Verpackung unmittelbar auf der Wunde aufgebracht wird, und die Blutung durch das vermehrte Angebot von biologisch aktivem Thrombin und Fibrinogen in kurzer Zeit, beispielsweise in etwa 2 min, zum Stillstand bringt.

Die mehrschichtige Ausbildung des Flachmaterials erlaubt eine räumliche Trennung von Fibrinogen und Thrombin und eine Anreicherung die Wundheilung fördernder, hier insbesondere das Fibroplasten-Wachstum fördernder Komponenten in wenigstens einer der Oberflächenschichten des Flachmaterials. Hieraus resultiert die hervorragende Lagerbeständigkeit ohne nennenswerte Abnahme der biologischen Aktivität. Ferner sind die Anforderungen an den Feuchtigkeitsausschluß geringer.

Weiterhin erlaubt es der mehrschichtige Aufbau, für das Thrombin und das Fibrinogen jeweils eine besonders angepaßte Umgebung vorzusehen. Das Thrombin befindet sich in derjenigen Außenschicht, die unmittelbar auf der Wunde aufgelegt wird, der sog. Kontaktschicht. Das Thrombin soll deshalb in einer Matrix verteilt sein, die schnell durchfeuchtbar ist und mechanisch stark belastbar ist, um die weiteren Schichten zu stützen. Als Matrixmaterial für die Thrombin-haltige Teilschicht kommen vorzugsweise Plasmaproteine (Albumin, Globuline) oder Fibrin-Spaltprodukte in Betracht. Das Fibrinogen andererseits soll in einer Matrix verteilt sein, die in das sich bildende Fibrinnetz einbaubar ist und physiologisch schnell abbaubar ist. Vorzugsweise sind deshalb als Matrixmaterialien für die Fibrinogenhaltige Teilschicht Glykoproteine wie Collagen, nicht vernetztes Fibrin oder Fibrinpolypeptide vorgesehen. Solche Substanzen weisen jedoch zumeist eine geringere mechanische Belastbarkeit, beispielsweise Reiß- oder Zugfestigkeit auf. Nach einem weiteren Gesichtspunkt der Erfindung ist deshalb vorgesehen, für die verschiedenen Teilschichten unterschiedliche Glykoproteine vorzusehen, die besonders an das jeweilige Anforderungsprofil angepaßt sind.

Dank der räumlichen Trennung ist das gemeinsame Aufbringen von Fibrinogen und Thrombin auf dem Trägermaterial nicht erforderlich. Sowohl Fibrinogen wie Thrombin lösen sich gut in Wasser oder überwiegend wässrigen Lösungen mit weiteren, mit Wasser mischbaren Lösungsmitteln. Das Aufbringen einer wässrigen, sowohl Fibrinogen wie Thrombin enthaltenden Lösung verbietet sich, da sich aus dem Fibrinogen Fibrin bilden und das letztere polymerisieren würde.

Verwendet man dagegen eine Suspension von Fibrinogen und Thrombin in einem organischen Lösungsmittel, so besteht die Gefahr einer teilweisen Denaturierung, die mit einem Aktivitätsverlust der Gerinnungsenzyme verbunden ist.

Der angegebene mehrschichtige Aufbau erlaubt es, genau definierte Fibrinogen- und Thrombinmengen sowie bestimmte Anzahlen an Fibroplasten-Zellen, von denen wenigstens ein Bruchteil reaktivierbar ist, pro Flächen- und/oder Volumeneinheit des Flachmaterials einzuhalten. Vorzugsweise wird das Fibrinogen oder Thrombin und gegebenenfalls die Fibroplasten-Zellen und/oder das die Fibroplasten-Einsprossung fördernde Mittel direkt in der zur Herstellung der jeweiligen Teilschicht dienenden, überwiegend wässrigen Lösung und/oder Suspension des Glykoproteins gelöst bzw. verteilt, so daß am fertigen Flachmaterial eine weitgehend homogene Verteilung von Fibrinogen bzw. Thrombin und der weiteren Komponenten in den jeweiligen Teilschichten gewährleistet ist. Diese Verteilung gewährleistet eine schnelle und hohe Wirksamkeit, die beispielsweise beim Aufbringen eines kristallinen, pulverförmigen Gemisches von Fibrinogen und Thrombin auf einem Träger nicht im gleichen Ausmaß zu erzielen ist.

Ferner erlaubt der mehrschichtige Aufbau die Anwendung reaktiver Zwischenschichten ohne besondere eigene mechanische Stabilität, weil die Außenschichten die Stabilität insbesondere Zugfestigkeit des Flachmaterials gewährleisten.

Schließlich gewährleistet der mehrschichtige Aufbau eine hohe Variationsbreite dahingehend, die Art, Anzahl und/oder Konzentration der verschiedenen Komponenten in den Teilschichten nach Bedarf einzustellen.

Das erfindungsgemäße Verfahren läßt sich höchst einfach durchführen, kommt mit überwiegend wässrigen Lösungen und/oder Suspensionen aus und vermeidet damit jeglichen Aktivitätsverlust von Fibrinogen und Thrombin sowie aktiven Fibroplasten-Zellen bei der Herstellung.

Die wesentlichen Komponenten für das erfindungsgemäße Flachmaterial sind bekannt und können nach bekannten Verfahren hergestellt, erzeugt und/oder im Fachhandel bezogen werden.

Das Flachmaterial besteht hauptsächlich aus Glykoprotein, welches die Flachmaterial-Teilschichten aufbaut und als Trägermaterial für die Gerinnungsenzyme dient. Das Glykoprotein soll vollständig resorbierbar und biologisch abbaubar sein, da es in das die Wunde schließlich bedeckende Fibrinnetzwerk eingebaut wird. Vorzugsweise sind solche Glykoproteine vorgesehen, von denen bereits aufgrund ihrer chemischen Natur und/oder der Oberflächenbeschaffenheit eine gewisse haemostatische Wirkung ausgeht, wie das beispielsweise für Collagen beschrieben ist. Tierisches Collagen ist wegen seiner nichtantigenen Eigenschaften besonders geeignet. Alle anderen Glykoproteine müssen dagegen aus humanem Material gewonnen werden. Das Trägermaterial soll beim Befeuchten mit dem Wundsekret die Flüssigkeit aufnehmen, sich anlösen und eine hochviskose, klebrige Paste bilden, die an der Wundfläche haftet, dem Ausströmdruck des Blutes standhält und die Gerinnungsenzyme des kontaktierenden Blutes aktiviert.

Vorzugsweise kommen im Rahmen der Erfindung als Glykoprotein nicht-vernetztes Fibrin, Fibrinspaltprodukte, Collagen, Globulin, Myoglobulin, Kasein oder Albumin in Betracht. In bestimmten Fällen wird eine Teilschicht auch aus zwei oder mehreren dieser Glykoproteine aufgebaut.

Unvernetztes Fibrin kann aus Faktor XIII-freiem Fibrinogen und Thrombin hergestellt werden. Ein gleiches Produkt erhält man bei der Umsetzung des Fibrinogens mit Calcium-freiem Thrombin und Cystein zur Blockierung der SH-Gruppen des Faktors XIII. Das entstehende Fibrin ist dann unvernetzt, kann ohne Faserbildung lyophilisiert und pulverisiert werden und kann in dieser Form zu einer Collagenlösung hinzugefügt werden.

Fibrin-Spaltprodukte werden durch den tryptischen Abbau von humanem Fibrin hergestellt. Das Material wird nach Proteolyse zu Pulver verarbeitet und kann in dieser Form eingesetzt werden. Auf diese Weise wird ein hoch-wasserlösliches Präparat erhalten.

Eine wässrige Lösung von Collagen tierischen Ursprungs kann im Fachhandel bezogen werden, beispielsweise von der Firma Pentapharm, Basel, Schweiz.

Geeignetes Globulin wird bei 50 %-iger Ammoniumsulfat-Konzentration (d. h. ein Teil Plasma auf ein Teil wässrige, gesättigte Ammoniumsulfat-Lösung) aus Humanplasma gefällt. Das Produkt wird abzentrifugiert, dialysiert und lyophilisiert.

Die weiteren geeigneten Glykoproteine wie Myoglobulin, Kasein und Albumin sind bekannte Substanzen und ebenfalls im Fachhandel zugänglich.

Besonders bevorzugt werden als Glykoproteine Collagen, Collagen-artige Materialien und Fibrin-Spaltprodukte, welche vorzugsweise das Gerüstmaterial für die Fibrinogen-haltige Teilschicht bilden, sowie nicht-vernetztes Fibrin und Fibrinpolypeptide, welche vorzugsweise das Gerüstmaterial für die Thrombin-haltige Teilschicht bilden.

Das Glykoprotein kann die Teilschicht in verschiedener Weise aufbauen. Verschiedene der genannten Glykoproteine fallen nach der herkömmlichen Aufbereitung bereits in einer blattartigen Struktur an, wie das insbesondere für Collagen und modifizierte Collagene beschrieben ist. Derartige Blatt-Strukturen können als Teilschicht des erfindungsgemäßen Flachmaterials dienen, nachdem sie mit einer wässrigen Lösung von Fibrinogen oder Thrombin getränkt worden sind. Eine weitere Möglichkeit besteht darin, das Glykoprotein zu verspinnen und aus den erhaltenen Fasern im Naß- oder Trockenverfahren ein Vlies zu erzeugen, das daraufhin mit der wässrigen Lösung von Fibrinogen oder Thrombin getränkt wird. Weiterhin kann die Teilschicht eine porige Schaumstruktur aufweisen. Hierzu wird das Glykoprotein in einer überwiegend wässrigen Lösung weitgehend gelöst. Die Erzeugung einer echten homogenen Lösung ist nicht erforderlich, vielmehr kann das Glykoprotein auch zu gelartigen oder gallertartigen Massen verarbeitet werden. Weiterhin können pulverförmige oder sonstige feinteilige Glykoproteine in wässriger Lösung aufgeschlämmt und/oder aufgequollen vorliegen. Als Lösungsmittel dient hauptsächlich Wasser oder ein Lösungsmittelgemisch, das neben geringeren Anteilen aus mit Wasser mischbaren organischen Lösungsmitteln wie etwa Dioxan, niedere Glykole, Äthanol, hauptsächlich aus Wasser besteht.

Eine solche überwiegend wässrige Lösung und/oder Suspension des Glykoproteins wird mit einer wässrigen Lösung des jeweiligen Gerinnungsproteins versetzt, gut durchmischt und das Gemisch daraufhin in dünner Schichtdicke in einer inerten Gießform tiefgefroren und lyophilisiert. Vor dem Tieffriervorgang kann das Gemisch mittels inerter Treibmittel ($N_2$, $CO_2$) verschäumt werden, wobei durch Zugabe oberflächenaktiver Mittel eine bestimmte Porengröße eingestellt werden kann. Für die Teilschicht(en) wird eine Schaumstruktur mit großem Oberflächenbereich erhalten. Sofern sämtliche Teilschichten aufgeschäumt werden, resultiert eine Verzahnung benachbarter Teilschicht-Oberflächen, was den Zusammenhalt und die Stabilität des Flachmaterials steigert. Vorzugsweise wird schrittweise nacheinander die gewünschte Anzahl Teilschichten aufgebaut, und der fertige Schichtenaufbau lyophilisiert. Das danach erhaltene Produkt läßt sich gut von der Formoberfläche abziehen, ist biegsam und ohne besondere Vorsichtsmaßnahmen handhabbar und kann leicht geschnitten, verpackt und sterilisiert werden. Um eine vorzeitige Aktivierung der Gerinnungsenzyme zu vermeiden, sollen die abschließenden Behandlungsschritte unter Feuchtigkeitsausschluß durchgeführt werden, so daß ein trockenes Flachmaterial erhalten und Feuchtigkeits-fest verpackt wird. Bei Bedarf kann innerhalb der Flachmaterial-Verpackung und mit der letzteren fest verbunden ein bekanntes Trocknungsmittel wie etwa Silikagel vorgesehen werden, um das Flachmaterial auch über eine längere Lagerdauer hinweg trocken zu halten.

Das erfindungsgemäße Flachmaterial ist vorzugsweise zur Anwendung am Menschen bestimmt. Das eingesetzte Fibrinogen ist deshalb vorzugsweise aus Human-Plasma gewonnen worden. Geeignete Präparate sind handelsüblich zugänglich und können beispielsweise von der Firma Bering-Werke, Marburg, bezogen werden. Ein gut geeignetes Präparat kann ferner entsprechend nachstehendem Verfahren isoliert werden.

Human-Plasma wird auf 4 °C gekühlt und unter Rühren mit β-Alanin (2 molare Lösung in Äthanol) versetzt, bis unter weiterer Athanol-Zugabe das Rohfibrinogen ausfällt. Dieses Rohfibrinogen wird abzentrifugiert, in 0,01 M Tris-Puffer (pH-Wert 7,4) gelöst und durch Zusatz von 2 M Glyzin erneut gefällt. Das abgetrennte Sediment wird in 0,9 %-iger wässriger NaCl-Lösung gelöst, gegen das gleiche Lösungsmittel dialysiert, entsalzt und anschließend lyophilisiert. Das erhaltene mikrokristalline Fibrinogen weist ein Molekulargewicht von 340 000 ± 5 % auf, ist in der α-Kette leicht angedaut, löst sich nach Einbringen in Körperflüssigkeit schnell, und beginnt unmittelbar darauf, beispielsweise in weniger als 2 min, zu polymerisieren. Der in Lösung gerinnbare Anteil des Fibrinogens macht wenigstens 85 % aus. 10 Gew.-Teile derartiges Fibrinogen enthalten weniger als 0,1 Gew.-Teile kälteunlösliches Globulin. Es ist festgestellt worden, daß die Fibrin-Polymerisation umso schneller verläuft, je weniger kälteunlösliches Globulin vorhanden ist. Derartiges, an kälteunlöslichem Globulin verarmtes Fibrinogen wird daher vorzugsweise eingesetzt.

Der Fibrinogen-Gehalt der Fibrinogen-haltigen Teilschicht kann 0,1 bis 30 mg, vorzugsweise 0,5 bis 10 mg pro 1 cm³ Glykoproteinmatrix betragen.

Das als weiteres notwendiges Gerinnungsenzym vorgesehene Thrombin dient als Startsubstanz für die Fibrinbildung und verkürzt die Reaktionszeit der Fibrinogen-Umwandlung im ausströmenden Blut. Das Thrombin soll unter bekannten, standardisierten Bedingungen wenigstens eine biologische Aktivität von 10.000 internationalen Einheiten/mg Thrombin aufweisen. Geeignete Präparate sind handelsüblich zugänglich. Beispielsweise kann geeignetes Thrombin in mikrokristalliner Form mit einer biologischen Aktivität von wenigstens 3 000 Einheiten/mg Präparat (das neben Thrombin bekannte Stabilisierungsmittel und Trägermaterialien enthält) unter der Handelsbezeichnung « Topostasin » von Hoffmann La Roche, Grenzach, Baden, bezogen werden. Weiterhin kann ein Teil des Thrombins durch Prothrombin ersetzt sein. Prothrombin bildet eine stabile, über lange Zeiträume lagerfähige Thrombinreserve, die bei Feuchtigkeitszutritt durch vorhandenes Thrombin und/oder das ausströmende Blut aktiviert wird. Prothrombin wird beispielsweise als PPSB-Präparat von der Firma Imuno-AG, Wien, vertrieben. Ein kombiniertes, Thrombin und Prothrombin enthaltendes Präparat kann beispielsweise durch Säulenchromatographie aus käuflichem Prothrombinkomplex abgetrennt werden oder aus Human-Plasma durch Bariumsulfat extrahiert und aus dem kristallinen Niederschlag rückgewonnen werden.

Der Thrombin-Gehalt einschließlich dem aus Prothrombin zur Verfügung stehenden Thrombin soll 10 bis 2 000 Einheiten, vorzugsweise 50 bis 1 000 Einheiten pro 1 cm³ Glykoproteinmatrix betragen. Diese Einheiten entsprechen den international gebräuchlichen NIH-Einheiten (National Institut of Health, U. S. A.).

Neben den Gerinnungsenzymen Fibrinogen und Thrombin kann das erfindungsgemäße Flachmaterial weitere auf die Blutgerinnung einwirkende Substanzen enthalten. Hierzu gehoren beispielsweise Fibrinolyse-Inhibitoren, welche die Wiederauflösung des bereits gebildeten Fibringerinnsels verhindern. Geeignete Fibrinolyse-Inhibitoren sind beispielsweise Antiplasmine wie Aprotenin, $\alpha_2$-Antiplasmin, $\alpha_2$Makroglobulin und/ oder Trypsin-Inhibitor. Weiterhin können Phospholipide, beispielsweise aus Hirnsubstanz oder Thrombozytenkonzentrat gewonnen, zugesetzt werden. Ferner kann zur gezielten Haemophilie-Behandlung ein erhöhtes Angebot der Faktoren VIII und/oder IX vorgesehen sein. Weiterhin können Faktoren zugesetzt werden, welche die Einsprossung und das Wachstum von Fibroplasten fördern und damit die Wundheilung beschleunigen. Zu diesem Zweck kann beispielsweise ein geringer Gehalt an Fibronektin vorgesehen sein.

Nach einem bevorzugten Gesichtspunkt der Erfindung enthält wenigstens eine Teilschicht des Flachmaterials - vorzugsweise eine oder beide Thrombin-haltige(n) Außenschicht(en) - in trockenem Zustand vorliegende Fibroplasten-Zellen, von denen wenigstens ein Bruchteil reaktivierbar ist. Fibroplasten sind langgestreckte, spindelförmige Zellen mit langen Zellfortsätzen. Sie kommen im lockeren Bindegewebe vor und bilden dort die collagenen sowie elastischen Fasern. Für die vorliegende Erfindung kommen Fibroplasten humanen Ursprungs in Betracht, die in geeigneten Nährmedien durch Zellteilung vermehrt worden sind. Beispielsweise können Fibroplasten aus juvenilem, mesenchymalem Gewebe isoliert und in Basalmedium nach « Eagle » der Firma Böhringer, Mannheim, mit Rinderserumalbumin-Zusatz durch Zellteilung leicht vermehrt werden. Aus der Kulturbrühe werden die Fibroplasten-Zellen durch Zentrifugieren abgetrennt und mit 0,9 % NaCl-Lösung gewaschen. Die in diesem Falle frisch angefallenen Fibroplasten-Zellen können daraufhin der ausgewählten Glykoprotein-Lösung und/oder -Suspension zur Erzeugung einer bestimmten Flachmaterial-Teilschicht zugesetzt werden. Alternativ können die frisch angefallenen Fibroplasten-Zellen in einem proteinhaltigen Lösungsmittel suspendiert, und diese Suspension bis zu ihrer Weiterverwendung tiefgefroren werden.

Die frisch angefallenen oder die in tiefgefrorenem Zustand gelagerten Fibroplasten-Zellen werden der zur Herstellung einer bestimmten Flachmaterial-Teilschicht dienenden Glykoprotein-Lösung und/oder -Suspension in einer solchen Menge zugesetzt, daß im fertigen trockenen Flachmaterial $10^3$ bis $10^{10}$ im trockenen Zustand vorliegende Fibroplasten-Zellen pro 1 cm³ Glykoprotein-Matrix vorhanden sind. Besonders bevorzugt wird ein Anteil von etwa $10^4$ bis $10^6$ in trockenem Zustand vorliegende Fibroplasten-Zellen pro 1 cm³ Glykoprotein-Matrix des fertigen trockenen Flachmaterials vorgesehen.

Im Rahmen der vorliegenden Erfindung ist festgestellt worden, daß wenigstens 10 %, zumeist 15 % und mehr, der nach dem Tieffrieren und Lyophilisieren in dem trockenen Flachmaterial im trockenen Zustand vorliegenden Fibroplasten-Zellen auch nach längerer Lagerung des Flachmaterials bei Raumtemperatur beim Kontakt mit Humanserum wieder reaktivierbar sind und in das Wundgebiet sowie in das mit dem Flachmaterial angebotene Substrat einsprossen. Soweit daher im Rahmen der vorliegenden Unterlagen (Patentbeschreibung und -Ansprüche) von « in trockenem Zustand vorliegenden Fibroplasten-Zellen » die Rede ist, soll der Ausdruck « in trockenem Zustand vorliegende Fibroplasten-Zellen » so verstanden werden und solche Randbedingungen umschreiben, etwa bei der Isolierung, Abtrennung und gegebenenfalls Lagerung der Fibroplasten-Zellen bei der Flachmaterial-Teilschicht-Herstellung und bei der Fertigstellung, Sterilisierung ! und Lagerung des trockenen Flachmaterials, daß aus der Gesamtzahl der vorhandenen trockenen Fibroplasten-Zellen wenigstens 10 % und vorzugsweise 15 % oder mehr beim Kontakt mit Humanserum wieder reaktivierbar sind.

Darüberhinaus ist festgestellt worden, daß auch die Anwesenheit von nicht-reaktivierbaren Fibroplasten-Zellen im erfindungsgemäßen Flachmaterial die Wundheilung positiv zu beeinflussen vermag vermutlich über das vermehrte Angebot bestimmter Proteine, Enzyme, Wirkstoffe und Faktoren.

Der Aufbau des interstitiellen Gewebes wird durch polyanionische Glykosaminglykane bewerkstelligt. Zu ihnen gehören die Chondroitinsulfate A, B und C (Chondroitinsulfat C = Dermatansulfat) und das Keratansulfat sowie die sulfatfreie Hyaluronsäure. Glykosaminglykane sind polymere Kohlehydrate (mittleres Molekulargewicht zumeist zwischen 6.000 und 12.000), die zusätzlich üblicherweise Sulfat- und Acetatgruppen enthalten. Diese Glykosaminglykane binden Proteine, so daß Proteoglykane entstehen, die ihrerseits Hyaluronsäure zu assoziieren vermögen. Auf diese Weise wird das körpereigene Bindegewebe gebildet, in das die Fibroplasten einsprossen.

Nach einem weiteren bevorzugten Gesichtspunkt der Erfindung ist in wenigstens einer Teilschicht des Flachmaterials ein Gehalt an diesen gezielt das Wachstum der Fibroplasten und deren Einsprossung fördernden Mitteln vorgesehen. Als solche Mittel kommen insbesondere Chondroitinsulfat, das handelsübliche Chondroitinsulfat-Gemisch (Chondroitinsulfat A und B), Dermatansulfat (Chondroitinsulfat C), Keratansulfat und Hyaluronsäure in Betracht. Besonders bevorzugt wird Chondroitinsulfat eingesetzt. Der Anteil an diesen das Wachstum der Fibroplasten und deren Einsprossung fördernden Mittel, insbesondere an Chondroitinsulfat, soll vorzugsweise 0,1 bis 1 mg pro 1 cm³ Glykoprotein-Matrix des fertigen trockenen Flachmaterials betragen. Die genannten Mittel wie Chondroitinsulfat, Chondroitinsulfat-Gemisch, Dermatansulfat, Keratansulfat und Hyaluronsäure sind handelsüblich zugänglich und können beispielsweise von den Firmen FLUKA Feinchemikalien GmbH, Neu-Ulm, BRD, oder SIGMA, München, BRD, bezogen werden.

Ein Gehalt an diesen, das Wachstum der Fibroplasten und deren Einsprossung fördernden Mittel, insbesondere Chondroitinsulfat, fördert das Wachstum und die Einsprossung der mit dem Serum im Wundgebiet angeschwemmten lebenden Fibroplasten und ist deshalb auch ohne zusätzliches Angebot an in trockenem Zustand vorliegenden Fibroplasten-Zellen zweckmäßig. Besonders bevorzugt wird jedoch die gemeinsame Anwesenheit an diesen, das Wachstum der Fibroplasten und deren Einsprossung fördernden Mittel, hier insbesondere Chondroitinsulfat, und von in trockenem Zustand vorliegenden Fibroplasten-Zellen im trockenen Flachmaterial — und hier vorzugsweise in wenigstens einer der Thrombin-haltigen Außenschichten vorgesehen.

Weiterhin können Salze zugesetzt werden, wie etwa NaCl, CaCl$_2$, Puffersalze wie Carbonat oder dergleichen, die in der Matrix verbleiben, die Gerinnungsfaktoren aktivieren und gegebenenfalls die Löslichkeit der Glykoproteine erhöhen.

Die Kontaktschicht, nämlich die Thrombin-haltige Teilschicht, enthält vorzugsweise zusätzlich Adrenalin und/oder Ergotamin. Beide Substanzen sind gefäßaktiv, was im Ergebnis zu einer schnelleren Blutgerinnung führt.

Vorzugsweise sind 0,05 bis 0,1 mg Adrenalin pro 1 cm³ Glykoprotein-Matrix vorhanden ; für das Ergotamin ist vorzugsweise in Anteil von 0,5 bis 10 µg pro 1 cm³ Glykoprotein-Matrix vorgesehen.

Sofern Antibiotika vorgesehen sind, wie beispielsweise Gentamycin (ein bekanntes Breitband-Antibiotikum) sollen diese vorzugsweise ebenfalls in der Thrombin-haltigen Teilschicht enthalten sein. Die Fibrinolyse-Inhibitoren können in allen Teilschichten vorhanden sein, befinden sich jedoch vorzugsweise in der Fibrinogen-haltigen Teilschicht.

Nachstehend wird die Erfindung im einzelnen anhand bevorzugter Ausführungsformen mit Bezugnahme auf die Figuren erläutert ; diese zeigen :

Fig. 1 eine zweischichtige Ausführungsform des Flachmaterials ; und

Fig. 2 eine dreischichtige Ausführungsform des Flachmaterials.

Nach einer Ausführungsform ist das erfindungsgemäße Flachmaterial zweischichtig ausgebildet und besteht aus einer Thrombin-freien Teilschicht, in deren Glykoprotein-Matrix das Fibrinogen weitgehend homogen verteilt ist, sowie aus der Fibrinogen-freien Teilschicht, in deren Glykoprotein-Matrix das Thrombin weitgehend homogen verteilt ist. Die Glykoprotein-Matrix der jeweiligen Teilschichten kann aus dem gleichen oder aus verschiedenen Glykoproteinen bestehen.

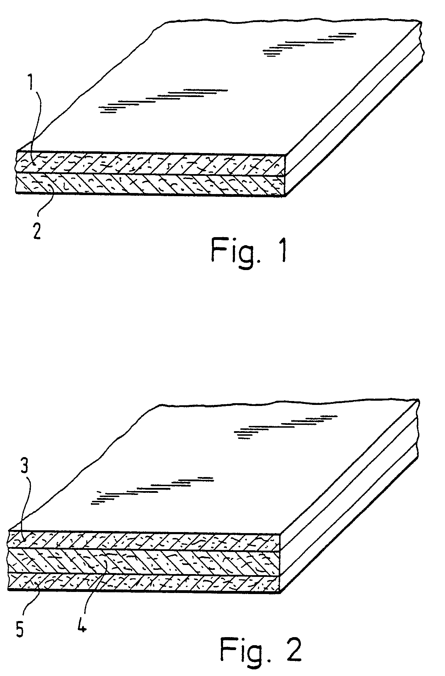

Eine solche Ausführungsform ist schematisch in Fig. 1 dargestellt, wobei die Thrombin-freie Teilschicht mit 1 und die Fibrinogen-freie Teilschicht mit 2 bezeichnet ist.

Bei einer solchen zweischichtigen Ausführungsform kann das Thrombin-Angebot gezielt an die zu versorgende Wundfläche angepaßt werden, beispielsweise für stark blutende Wunden ein hohes Trombin-Angebot zur schnellen Blutstillung bei geringem zusätzlichem Fibrinogen-Angebot vorgesehen werden.

Die Thrombin-haltige Teilschicht ist zur unmittelbaren Auflage auf der Wunde bestimmt und wird hierzu zweckmäßigerweise markiert, beispielsweise mit Haemoglobin angefärbt.

Vorzugsweise weist jede Teilschicht 1 und 2 eine Schaum- oder Vliesstruktur auf. Dieser Aufbau gewährleistet dank seiner großen Oberfläche eine gute Saugfähigkeit. Die erhöhte Kontaktfläche aktiviert die im anströmenden Blut enthaltenen Gerinnungsfaktoren, sowie die mit den Flachmaterial zusätzlich angebotenen Faktoren. Ein aus Glykoprotein-Fasern aufgebautes Vlies weist eine noch größere mechanische Stabilität als eine entsprechende Schaumstruktur auf ; dies kann beispielsweise für die Versorgung von Muskelrissen wichtig sein.

Für jede Teilschicht des zweischichtigen Aufbaus ist vorzugsweise eine Schichtdicke von etwa 1 bis 5 mm vorgesehen. Diesen Schichtdickenbereich kann das anströmende Blut schnell durchfeuchten, so daß es zu einer raschen Aktivierung der mit dem Flachmaterial angebotenen Gerinnungsfaktoren kommt.

Nach einer weiteren Ausführungsform kann das erfindungsgemäße Flachmaterial dreischichtig aufgebaut sein. Eine solche Ausführungsform ist schematisch in Fig. 2 dargestellt, wobei die mittig angeordnete Teilschicht mit 4, sowie die beiden außen liegenden Teilschichten mit 3 und 5 bezeichnet sind.

Bei einem solchen dreischichtigen Aufbau enthält vorzugsweise die mittig angeordnete Teilschicht 4 das Fibrinogen und wenigstens eine der beiden außen liegenden Teilschichten 3 und 5 das Thrombin. Für manche Anwendungsfälle, beispielsweise bei Flachmaterialien, die im Bauchraum eingesetzt werden sollen, etwa zur Versorgung von Darmnähten, kann es zweckmäßig sein, die mittig angeordnete, Fibrinogen-haltige Teilschicht auf einer Seite mit einer Thrombin-haltigen Teilschicht zu belegen und auf der anderen Seite mit einer sowohl Thrombin- wie Fibrinogen-freien Teilschicht zu belegen, die jedoch nicht-gerinnungsaktive Proteine wie Albumin oder Globulin enthält. Damit wird sicher verhindert, daß die sich bildenden Fibrinnetze zu einer Verklebung der Darmschlingen führen.

Bei einer weiteren Ausführungsform des dreischichtigen Flachmaterials ist in beiden außen liegenden Teilschichten Thrombin enthalten. Diese Ausführungsform eignet sich vor allem zum Versorgen von tiefergehenden Muskelläsionen oder Rupturen weicher Organe (Leber, Milz, Pankreas), weil die beidseitige Thrombinschicht schnell für den Verschluß der vielen eröffneten Kapillaren in den erwähnten Organen sorgt. Auch bei stärker blutenden Operationswunden, wie sie in der Gynäkologie oder Orthopädie häufig auftreten, sind diese Thrombin-haltigen Wundmaterialien vorteilhaft einsetzbar. Die Thrombin-haltigen, zum unmittelbaren Kontakt mit der Wunde bestimmten Teilschichten müssen nicht besonders markiert werden. Beim Einlegen in einen Wundspalt reagieren beide Flachmaterial-Oberflächen mit der angrenzenden Wundfläche.

Beim dreischichtigen Aufbau wird für die mittig angeordnete Teilschicht 4 vorzugsweise eine größere Schichtdicke vorgesehen, als für die beiden außen liegenden Teilschichten 3 und 5. Dies erlaubt den Aufbau der mittig angeordneten, Fibrinogen-haltigen Teilschicht 4 aus solchen Glykoproteinen, welche unmittelbar in das Fibrin-Netzwerk eingebaut werden und in der Regel nur eine geringe mechanische Festigkeit aufweisen, wie beispielsweise Fibrin-Spaltprodukte. Auf diese Weise kann die mechanische Belastbarkeit des Wundverschlusses gesteuert werden. Durch die zusätzliche Anwesenheit von Collagen und/oder modifizierten Collagenen kann die mechanische Festigkeit weiter gesteigert werden. Je mehr Fibrinogen pro Flächeneinheit mit der mittigen Teilschicht 4 angeboten wird, desto mehr Fibrin wird gebildet, was wiederum die mechanische Belastbarkeit des Wundverschlusses erhöht.

Nach einer weiteren Ausführungsform der Erfindung kann die in Fig. 2 dargestellte einschichtige mittige Teilschicht 4 durch zwei oder mehr Einzelschichten ersetzt sein. Es resultiert dann ein vier- oder noch höherschichtiger Aufbau, wobei die Zusammensetzung der innen liegenden Teilschichten an bestimmte Anwendungen angepaßt werden kann, beispielsweise eine verzögerte Wirkstoffabgabe und damit eine verlängerte Einwirkung auf die Wundheilung erreicht werden kann. Beispielsweise können eine Anzahl zwischen den äußeren Kontaktschichten befindliche Teilschichten mit unterschiedlichem Wirkstoffgehalt vorgesehen werden, die bei ihrer Auflösung ihre Inhaltsstoffe in zeitlich abgestufter Reihenfolge in das Wundgebiet abgeben. Eine solche Ausführungsform empfiehlt sich beispielsweise zur Versorgung infizierter Knochenbrüche, denen über längere Zeit Antibiotika zugeführt werden müssen, die dann durch die unterschiedlichen Abbaugeschwindigkeiten der Teilschichten zeitlich gestaffelt freigesetzt werden.

Für den optimalen Aufbau des erfindungsgemäßen Flachmaterials gelten insbesondere folgende Gesichtspunkte :

Die Auflösungsgeschwindigkeit des Glykoprotein-Trägermaterials bestimmt das Einsatzgebiet des Fibrinogenvlieses.

Wird eine lange Verweildauer des Wundverschlußmaterials gewünscht, muß Collagen als Trägermaterial eingesetzt werden. Soll nur ein rascher Wundverschluß erzielt, das Material aber rasch durch körpereigene Proteasen abgebaut werden, muß auf andere Plasmaproteine (Albumin, Globulin, Fibrin) ausgewichen werden.

Ein optimaler Aufbau soll daher mit Thrombin-haltigen Außenschichten beginnen.

Dafür eignet sich Albumin oder Globulin als Träger (in Trockenform).

Geringe Zusätze von Collagen erhöhen in dieser Schicht die mechanische Stabilität.

Ein zusätzlicher Gehalt an in trockenem Zustand vorliegenden Fibroplasten-Zellen und/oder von das Wachstum der Fibroplasten und deren Einsprossung fördernden Mittel, und hier insbesondere Chondroitinsulfat in wenigstens einer der Thrombin-haltigen Außenschichten fördert die Wundheilung.

Die Fibrinogen-haltige Schicht soll mittig angeordnet sein, während die folgende Außenseite nur Thrombin enthalten darf oder abdeckende, nicht gerinnungsaktive Proteine wie Albumin oder Globulin.

Bei einer besonders bevorzugten Ausführungsform des Flachmaterials besteht eine Außenschicht aus einer Thrombin-haltigen Schicht (hergestellt aus einer 200 bis 1 000 Einheiten Thrombin pro 1 ml enthaltenden wässrigen 5 %-igen Albumin-Lösung) mit einer Dicke von etwa 2 mm. Der nachfolgende Collagenträger enthält das Fibrinogen. Diese Schicht wird durch Tieffrieren aus wässriger, salzhaltiger Collagen-Lösung mit 1 mg Fibrinogen pro ml Lösung erhalten. Der zumeist im Bereich von 0,8 bis 2,0 % liegende Collagengehalt kann bis auf eine Endkonzentration von 0,25 % abgesenkt werden, sofern zusätzlich eine Fibrinsuspension oder ein anderes Plasmaprotein als Stabilisierungsmittel zugegeben wird. Die zweite Außenschicht wird durch Albumin aus 5 %-iger wässriger Lösung gebildet.

Bei Bedarf wird auch dieser Außenschicht Thrombin zugesetzt.

Die nachstehenden Beispiele dienen zur weiteren Erläuterung der Erfindung, ohne diese einzuschränken.

Beispiel 1 :

Eine handelsüblich zugängliche (Fa. Pentapharm, Basel, Schweiz) Collagen-Lösung, enthaltend 1 % Collagen in salzhaltigem Wasser, wird mit 0,5 mg festem, mikrokristallinem Fibrinogen (nach dem oben beschriebenen Verfahren erhalten) sowie mit 10 mg festem Albumin, jeweils pro 1 ml Lösung, versetzt. Man rührt bei Raumtemperatur einige Minuten, bis sich Fibrinogen und Albumin vollständig aufgelöst haben.

Diese Lösung wird in eine flache inerte Schale mit ebenem Boden gegossen, bis man eine 3 mm starke Flüssigkeitsschicht erhält. Daraufhin wird tiefgefroren, wozu man die Schale mit Inhalt etwa 45 min lang bei — 40 °C hält.

Daraufhin wird auf die Oberfläche der gebildeten Eisschicht soviel Thrombin-haltige Collagen-Lösung aufgegossen, daß sich erneut eine etwa 2 mm starke Flüssigkeits-Eisschicht bildet. Zum Aufgießen weist diese zweite Lösung zweckmäßigerweise Zimmertemperatur auf. Dadurch schmilzt die zuvor gebildete Eisschicht oberflächlich und nach dem erneuten Tieffrieren, gefolgt von einer Lyophilisation, wird eine sehr stabile Schichtenverbindung erhalten, ohne daß es wegen der niedrigen Temperaturen und der kurzen Reaktionsdauer bereits zu einer nennenswerten Umsetzung des Fibrinogens kommt. Zur Bildung dieser zweiten Lösung ist Thrombin (« Topostasin » von Hoffmann La Roche, Grenzach, Baden) in der oben genannten (jedoch Fibrinogen-freien) Collagen-Lösung aufgelöst worden (200 Einheiten Thrombin pro 1 ml Lösung). Es wird erneut tiefgefroren, wozu man etwa 45 min lang bei — 40 °C hält. Daraufhin wird unter üblichen Bedingungen lyophilisiert.

Nach Abschluß des Trocknungsverfahrens wird das Flachmaterial vorsichtig an einem Rand vom Formboden gelöst und daraufhin in Form eines Lappens abgezogen. Man erhält ein großflächiges, biegsames, flexibles Proteinvlies mit einer Schichtdicke von etwa 5 mm. Der Lappen wird kurzzeitig auf einer Al-Folie abgelegt, auf die gewünschten Maße zurechtgeschnitten, die erhaltenen Teilstücke in eine eingesenkte Plastikform eingelegt, und die letztere mit einer Al-Folie verschlossen. Daraufhin wird die Verpackung samt Inhalt mit Röntgenstrahlung (Dosis : 3 min lang 3 000 rad) sterilisiert.

Das erhaltene Produkt ist bei Raumtemperatur praktisch unbegrenzt lagerfähig, ohne nennenswert an Aktivität zu verlieren. Zur Anwendung wird die Packung geöffnet und das zweischichtige Flachmaterial mit der Thrombin-haltigen Teilschicht unmittelbar auf die blutende Wunde aufgelegt und leicht angedrückt. Das Flachmaterial resorbiert das aus der Wunde ausgetretene Blut und/oder Plasma rasch und bringt es sehr schnell zum Gerinnen — je nach Blutaustritt in der Wundfläche auf jeden Fall innerhalb von 3 bis 5 min. Dabei färbt sich das Flachmaterial rot und bildet auf der Wundfläche einen stabilen Fibrin/Collagen-Kuchen.

Beispiel 2 :

Ein weiteres, zweischichtiges Flachmaterial wird im wesentlichen analog zu Beispiel 1 erzeugt. Abweichend dient zur Bildung der Thrombin-haltigen Teilschicht eine wässrige, salzhaltige (0,9 %-ig an NaCl, 0,025 molar an $CaCl_2$) 5 %-ige Albumin-Lösung, die pro 1 ml mit 100 Einheiten Thrombin (Topostasin®), 50 Einheiten Prothrombin (PPSB-Präparat der Fa. Immero AG, Wien), 1 000 Einheiten Gentamycin als Antibiotikum versetzt wird. Ferner werden der gesamten Lösung einige Kristalle Haemoglobin zugesetzt, um die Kontaktschicht zu markieren. Diese Lösung wird bis zu einer Schichtdicke von 2 mm in die Form gegossen und tiefgefroren (— 40 °C). Auf der erhaltenen Eisschicht wird eine 1 %-ige wässrige Collagen-Lösung mit 3 mg Fibrinogen pro 1 ml bis zu einer Schichtdicke von 3 mm aufgegossen, erneut tiefgefroren und daraufhin lyophilisiert.

Beispiel 3 :

Die Erzeugung eines dreischichtigen Flachmaterials erfolgt im wesentlichen analog zu Beispiel 1. Eine wässrige 3 %-ige Albumin-/0,5 %-ige Collagen-Lösung wird pro 1 ml mit 50 Einheiten Thrombin versetzt. Die Lösung wird bis zu einer Schichthöhe von 2 mm in eine Form mit inerter Oberfläche gegossen und tiefgefroren (— 40 °C). Auf die gefrorene Schicht wird eine 1 %-ige Collagen-Lösung mit 3 mg Fibrinogen und 2 mg Aprotenin (als Fibrinolyse-Inhibitor) pro 1 ml Lösung bis zu einer Schichtdicke von 4 mm aufgegossen und ebenso tiefgefroren. Als Abschluß wird wieder bis zu einer Schichtdicke von 4 mm obige Albumin-/Collagen-/Thrombin-Lösung aufgetragen und nach dem Tieffrieren die dreifach beschichtete Form gefriergetrocknet. Das gebildete trockene Flachmaterial wird unter Feuchtigkeitsausschluß an einer Seite angeboben, mit einem stumpfen Spatel von der Form gelöst und nach Zuschneiden in die angegebene Größe in die Verpackungsfolie eingeschweißt und die Verpackung samt Inhalt sterilisiert.

Beispiel 4 :

Thrombin wird in eine 5 %-ige Human-Globulin-Lösung (1 000 Einheiten Thrombin pro 1 ml Lösung) eingebracht und diese Lösung anschließend mit Stickstoffgas verschäumt. Das schaumige Material wird in die Gefriertrocknungsform eingegossen und tiefgefroren. Die Schichthöhe beträgt ungefähr 3 mm. Auf die Thrombin-haltige Schicht wird eine Fibrinogen-haltige Collagen-Lösung (10 mg Fibrinogen und 0,075 mg Adrenalin (käufliches Präparat) in jeweils 1 ml 1 %-iger Collagen-Lösung) bis zu einer Schichthöhe von 5 mm ohne Verschäumen aufgetragen und ebenfalls tiefgefroren. Die Endschicht wird aus obiger Thrombin-haltiger 5 %-iger Human-Globulin-Lösung gebildet, die als Schaum bis zu einer Gesamthöhe von 8 mm aufgetragen wird. Nach dem Lyophilisieren wird das Material formgerecht geschnitten und verpackt. Nach dem Verpacken wird strahlen-sterilisiert.

Beispiel 5 :

Unvernetztes Fibrin wird zu einem Pulver verarbeitet und in einem Anteil von 0,6 g pro 1 ml in Wasser suspendiert. Als Zusatz wird 1 % Human-Albumin zugegeben. Thrombin wird in einer Menge von 300 Einheiten pro 1 ml zugesetzt. Die Fibrin-Albumin-Thrombin-Suspension wird in eine Gießform bis zu einer Höhe von 2 mm gegossen und sofort tiefgefroren.

Auf die Eisschicht wird eine 1 %-ige Collagen-Lösung mit 1 mg Fibrinogen pro 1 ml und 1 mg Fibrinpartikeln pro 1 ml bis zu einer Schichtdicke von 4 mm eingegossen.

Auf die tiefgefrorene zweite Schicht wird als Abschluß obige Thrombin-haltige Albumin-Fibrin-Suspension aufgetragen, tiefgefroren und das mehrschichtige Gefriermaterial lyophilisiert. Die ablösbare Protein-Trockenmasse bildet ein stabiles Flachmaterial, das sich leicht schneiden läßt.

Bei der Benutzung als Wundauflagematerial ist es gut manipulierbar, läßt sich biegen, was besonders bei der Versorgung von Darmnähten erforderlich ist, und saugt Blut oder Plasma im Wundgebiet schnell auf.

Beispiel 6 :

Zur Erzeugung eines vierschichtigen Flachmaterials — im wesentlichen analog zu Beispiel 1 — dienen die nachstehenden Lösungen/Suspensionen :

a) 50 Einheiten Thrombin pro 1 ml einer 5 %-igen wässrigen, salzhaltigen Albumin-Lösung ;

b) 50 Einheiten Prothrombin pro 1 ml einer 2,5 %-igen, wässrigen Albumin-Lösung ;

c) 1 mg Fibrinogen, 5 000 Einheiten Gentamycin, 8 μg Ergotamin, 10 000 E. $\alpha_2$-Antiplasmin, 10 000 E. $\alpha_2$-Makroglobulin pro 1 ml 5 %-iger wässriger Albumin-Lösung ;

d) 2,0 %-ige wässrige Collagen-Lösung mit Zusatz von 5 000 Einheiten Gentamycin pro 1 ml Lösung.

Zur Herstellung des Flachmaterials wird die Lösung « a » bis zu einer Schichtdicke von 2 mm in eine Form mit inerter Oberfläche eingegossen und tiefgefroren. Darauf wird die Lösung « b » bis zu einer Schichtdicke von 2 mm, die Lösung « c » bis zu einer Schichtdicke von 2 mm und abschließend die Lösung « d » bis zu einer Schichtdicke von 4 mm eingegossen, nachdem die vorausgegangene Schicht jeweils tiefgefroren war. Das fertige aufgetaute trockene Präparat weist eine weitgehend flächenstabile, zusammendrückbare Vliesstruktur mit einer Gesamtschichtdicke von etwa 10 mm auf. Die Mehr-Schichtenbildung erlaubt das Einbringen verschiedener Reaktionspartner oder Pharmaka, so daß bei der Versorgung infektiösen Wundflächen die Abgabe der Antiobiotika verzögert erfolgt und einen längeren Infektionsschutz gewährleistet. Collagen der löslichen Form, wie es bei der Herstellung dieses Produktes verwandt wird, bleibt im abheilenden Wundgebiet für mehr als 14 Tage nachweisbar. Die im Verbund mit dem Flachmaterial angebotenen Pharmaka diffundieren in dieser Zeit in das umgebende Gewebe. Die Diffusionsgeschwindigkeit wird von der Assoziation z. B. des Antibiotikums zum Collagenträger oder Fibrin bestimmt.

Beispiel 7 :

Im wesentlichen wird das Beispiel 1 wiederholt ; abweichend werden der Thrombin-haltigen Collagen-Lösung zusätzlich Fibroplasten-Zellen zugesetzt.

Aus juvenilem, mesenchymalen Humangewebe stammende Fibroplasten-Zellen werden in Basalmedium nach « Eagle » der Fa. Böhringer, Mannheim, BRD, dem zusätzlich Rinderserumalbumin zugesetzt worden war, gezüchtet. Diese Fibroplasten-Zellen werden durch Zentrifugieren aus ihrem Nährmedium abgetrennt und mit 0,9 % NaCl-Lösung gewaschen und danach in 5 %-iger Humanalbumin-Lösung aufgenommen und ohne Zerstörung der Zellen suspendiert.

Der in Beispiel 1 verwendeten, durch Auflösen von « Topostasin » in salzhaltiger 1 %-iger Collagen-Lösung gewonnenen Lösung wird ein solcher Anteil dieser Fibroplasten-Suspension zugesetzt, daß die Thrombin-haltige Collagen-Lösung $10^3$ bis $10^6$ Fibroplasten-Zellen pro 1 ml Collagen-Lösung enthält. Anschließend wird diese Thrombin und Fibroplasten enthaltende Collagen-Lösung auf die bereits gebildete Eisschicht aus Fibrinogen-haltiger Collagen-Lösung aufgegossen und ohne Verschäumen tiefgefroren. Anschließend werden beide Schichten gemeinsam lyophilisiert, geschnitten, verpackt und sterilisiert. Um die Fibroplasten-Zellen nicht zu schädigen, erfolgt die Sterilisierung bei schwächerer Dosis über eine längere Zeitspanne, beispielsweise 9 min lang 1 000 rad.

Beispiel 8 :

Im wesentlichen wird das Beispiel 7 wiederholt ; abweichend wird der Thrombin und Fibroplasten enthaltenden Lösung zusätzlich Chondroitinsulfat zugesetzt.

Im einzelnen wird eine salzhaltige, 1 %-ige Collagen-Lösung mit durch Abzentrifugieren aus ihrer Kulturbrühe erhaltenen Fibroplasten-Zellen so angereichert, daß 1 ml Lösung etwa $10^3$ Fibroplasten-Zellen enthält. Danach wird handelsübliches Chondroitinsulfat-Gemisch (Chondroitinsulfat A und B, mittleres Molekulargewicht etwa 8 000, bezogen von der Fa. FLUKA, Feinchemikalien GmbH, Neu-Ulm, BRD) in einer Menge von 1 mg pro 1 ml Lösung zugesetzt und aufgelöst. Danach werden 30 Einheiten Thrombin pro 1 ml Lösung zugesetzt.

Diese Lösung wird in einer Schichtdicke von 3 mm auf einer vorgebildeten Eisschicht aus Fibrinogen-haltiger Collagen-Lösung (3 mg Human-Fibrinogen pro 1 ml salzhaltiger, 1 %-iger Collagen-Lösung) aufgebracht und tiefgefroren. Anschließend werden beide Schichten gemeinsam lyophilisiert, geschnitten, verpackt und sterilisiert.

Beispiel 9 :

Im wesentlichen analog zu Beispiel 7 wird ein dreischichtiges Flachmaterial erzeugt.

Fibroplasten-Zellen werden durch Zentrifugieren aus ihrer Kulturbrühe abgetrennt und in einer salzhaltigen, 1 % Albumin-Lösung suspendiert. Nach Einstellen einer Konzentration von $10^5$ Fibroplasten-Zellen auf 1 ml Lösung werden 0,3 mg Chondroitinsulfat und 300 Einheiten Thrombin — jeweils auf 1 ml Lösung — zugesetzt. Die erhaltene erste Suspension wird bis zu einer Schichtdicke von 4 mm in eine flache, 3,5 × 8 cm große Form gegossen und darin tiefgefroren.

Zur Erzeugung der mittleren Schicht wird Fibrinogen (5 mg pro 1 ml Lösung) in salzhaltiger, 1 %-iger Collagen-Lösung aufgenommen. Diese zweite Lösung wird auf die Eisschicht aus der ersten Suspension aufgegossen und ebenfalls tiefgefroren.

Zur Erzeugung der anderen Außenschicht wird erneut die oben genannte erste, Fibroplasten-Suspension in Thrombin und Chondroitinsulfat enthaltender Albumin-Lösung verwendet.

Nach dem erneuten Tieffrieren und der gemeinsamen Lyophilisierung wird ein dreischichtiges Flachmaterial mit einer Gesamtschichtdicke von 12 mm erhalten.

**Patentansprüche**

1. Resorbierbares Flachmaterial zum Abdichten und Heilen von Wunden, im wesentlichen bestehend aus einer Glykoprotein-Matrix mit einem Gehalt an die Blutgerinnung bewirkenden bzw. fördernden Substanzen einschl. Fibrinogen und Thrombin, dadurch gekennzeichnet, daß das Flachmaterial trocken und mehrschichtig ausgebildet ist ; wenigstens eine Teilschicht (1, 4) Thrombin-frei ist und das Fibrinogen in deren Glykoprotein-Matrixweitgehend homogen verteilt enthält ; und wenigstens eine weitere Teilschicht (2, 3) Fibrinogen-frei ist und das Thrombin in deren Glykoprotein-Matrix weitgehend homogen verteilt enthält.

2. Flachmaterial nach Anspruch 1, dadurch gekennzeichnet, daß wenigstens eine Teilschicht (1, 2, 3 und/oder 4) in trockenem Zustand vorliegende Fibroplasten-Zellen enthält.

3. Flachmaterial nach Anspruch 1 oder 2, dadurch gekennzeichnet, daß wenigstens eine Teilschicht (1, 2, 3 und/oder 4) — neben gegebenenfalls Thrombin — ein gezielt das Wachstum und/oder die Einsprossung von Fibroplasten förderndes Mittel enthält.

4. Flachmaterial nach Anspruch 3, dadurch gekennzeichnet, daß das gezielt das Wachstum und/oder die Einsprossung von Fibroplasten fördernde Mittel Chondroitinsulfat ist.

5. Flachmaterial nach einem der Ansprüche 1 bis 4, dadurch gekennzeichnet, daß das Flachmaterial zweischichtig ausgebildet ist.

6. Flachmaterial nach Anspruch 5, dadurch gekennzeichnet, daß jede Teilschicht (1, 2) des zweischichtigen Flachmaterials (Fig. 1) eine Schaum- oder Vliesstruktur aufweist ; und jede Teilschicht (1, 2) eine Schichtdicke zwischen 1 und 5 mm aufweist.

7. Flachmaterial nach einem der Ansprüche 1 bis 4, dadurch gekennzeichnet, daß das Flachmaterial dreischichtig angeordnet ist.

8. Flachmaterial nach Anspruch 7, dadurch gekennzeichnet, daß die mittig angeordnete Teilschicht (4) des dreischichtigen Flachmaterials (Fig. 2) Thrombin-frei ist und das Fibrinogen weitgehend homogen verteilt enthält ;

jede außen liegende Teilschicht (3, 5) Fibrinogen-frei ist ; und wenigstens eine außen liegende Teilschicht (3, 5) das Thrombin weitgehend homogen verteilt enthält.

9. Flachmaterial nach Anspruch 7 oder 8, dadurch gekennzeichnet, daß die Fibroplasten-Zellen und/oder das gezielt das Wachstum und/oder die Einsprossung von Fibroplasten fördernde Mittel, hier insbesondere Chondroitinsulfat, in wenigstens einer der außen liegenden Teilschichten enthalten sind.

10. Flachmaterial nach einem der Ansprüche 7 bis 9, dadurch gekennzeichnet, daß das Glykoprotein der mittig angeordneten Teilschicht (4) sich von demjenigen der außen liegenden Teilschichten (3, 5) hinsichtlich seiner chemischen Natur oder bei gleichem Glykoprotein hinsichtlich seiner Dichte, seiner Schichtdicke, seines Polymerisationsgrades und/oder seiner mechanischen Belastbarkeit unterscheidet.

11. Flachmaterial nach einem der Ansprüche 7 bis 10, dadurch gekennzeichnet, daß die mittig angeordnete Teilschicht (4) des dreischichtigen Flachmaterials eine größere Schichtdicke aufweist als dessen außen liegende Teilschichten (3, 5).

12. Flachmaterial nach einem der Ansprüche 7 bis 11, dadurch gekennzeichnet, daß alle Teilschichten (3, 4, 5) des dreischichtigen Flachmaterials eine Schaum- oder Vliesstruktur aufweisen ;

die mittig angeordnete Teilschicht (4) eine Schichtdicke zwischen 3 und 8 mm aufweist ; und die außen liegenden Teilschichten (3, 5) je eine Schichtdicke von 1 bis 3 mm aufweisen.

13. Flachmaterial nach einem der Ansprüche 1 bis 12, dadurch gekennzeichnet, daß die Thrombin-haltige Teilschicht 10 bis 2 000 Einheiten Thrombin pro 1 cm³ Glykoprotein-Matrix enthält.

14. Flachmaterial nach einem der Ansprüche 1 bis 13, dadurch gekennzeichnet, daß die Thrombin-haltige Teilschicht zusätzlich gefäß-aktivierend wirkende Substanzen enthält, insbesondere Adrenalin und/oder Ergotamin.

15. Flachmaterial nach einem der Ansprüche 2 bis 14, dadurch gekennzeichnet, daß die Fibroplasten enthaltende Teilschicht 10³ bis 10¹⁰ Fibroplasten-Zellen pro 1 cm³ Glykoprotein-Matrix enthält.

16. Flachmaterial nach Anspruch 15, dadurch gekennzeichnet, daß die Fibroplasten enthaltende Teilschicht zusätzlich 0,1 bis 1 mg Chondroitinsulfat pro 1 cm³ Glykoprotein-Matrix enthält.

17. Flachmaterial nach einem der Ansprüche 1 bis 16, dadurch gekennzeichnet, daß die Fibrinogen-haltige Teilschicht 0,1 bis 30 mg Fibrinogen pro 1 cm³ Glykoprotein-Matrix enthält.

18. Flachmaterial nach einem der Ansprüche 1 bis 17, dadurch gekennzeichnet, daß das Flachmaterial eine Länge von 3 bis 12 cm, eine Breite von 1 bis 12 cm und eine Gesamt-Schichtdicke von 5 bis 20 mm aufweist.

19. Flachmaterial nach einem der Ansprüche 1 bis 18, dadurch gekennzeichnet, daß das Glykoprotein nicht-vernetztes Fibrin, ein Fibrinspaltprodukt, Collagen, Globulin, Myoglobulin, Kasein oder Albumin oder ein Gemisch aus zwei oder mehr dieser Komponenten ist.

20. Flachmaterial nach einem der Ansprüche 1 bis 19, dadurch gekennzeichnet, daß die Glykoprotein-Matrix durch Verschäumen und/oder Tieffrieren einer weitgehend homogenen, überwiegend wässrigen Glykoprotein-Lösung und/oder -Suspension und anschließender Lyophilisation erhalten worden ist.

21. Flachmaterial nach einem der Ansprüche 1 bis 20, dadurch gekennzeichnet, daß die Glykoprotein-Matrix ein Vlies bildet, dessen Fasern durch Verspinnen einer homogenen, überwiegend wässrigen Glykoprotein-Lösung erhalten worden sind.

22. Verfahren zur Herstellung eines Flachmaterials nach einem oder mehreren der Ansprüche 1 bis 21, dadurch gekennzeichnet, daß eine Thrombin-freie, überwiegend wässrige Lösung und/oder Suspension des Glykoproteins mit Fibrinogen versetzt und zu einer Flachmaterial-Teilschicht verarbeitet wird ; und auf dieser Teilschicht wenigstens eine weitere Teilschicht vollflächig aufgebracht wird, die ihrerseits aus einer weiteren, Fibrinogen-freien und Thrombin-haltigen Lösung und/oder Suspension des nämlichen oder eines anderen Glykoproteins erhalten worden ist.

23. Verfahren nach Anspruch 22, dadurch gekennzeichnet, daß die Thrombin-haltige Lösung oder Suspension des Glykoproteins zusätzlich mit aktiven Fibroplasten-Zellen angereichert wird.

24. Verfahren nach Anspruch 22 oder 23, dadurch gekennzeichnet, daß die Thrombin-haltige Lösung oder Suspension des Glykoproteins zusätzlich mit einem das Wachstum und/oder die Einsprossung von Fibroplasten förderndem Mittel angereichert wird.

25. Verfahren nach Anspruch 24, dadurch gekennzeichnet, daß der Thrombin-haltigen Lösung oder Suspension des Glykoproteins zusätzlich Chondroitinsulfat zugesetzt wird.

26. Verfahren nach einem der Ansprüche 22 bis 25, dadurch gekennzeichnet, daß zur Erzeugung der Flachmaterial-Teilschicht die jeweilige Lösung und/oder Suspension auf einer inerten Oberfläche aufgebracht, dort tiefgefroren und schließlich lyophilisiert wird.

27. Verfahren nach einem der Ansprüche 22 bis 26, dadurch gekennzeichnet, daß die Teilschichten unabhängig voneinander erzeugt werden ;

wenigstens eine Teilschicht-Oberfläche mit Wasser angefeuchtet wird ; und eine weitere Teilschicht anderer Zusammensetzung auf der angefeuchteten Teilschicht-Oberfläche aufgelegt wird.

28. Verfahren nach einem der Ansprüche 22 bis 26, dadurch gekennzeichnet, daß eine Lösung und/oder Suspension einer Zusammensetzung auf einer inerten Oberfläche aufgebracht und dort tiefgefroren wird ; und auf der erhaltenen Eisschicht-Oberfläche noch im tiefgefrorenen Zustand eine Lösung und/oder Suspension anderer Zusammensetzung aufgebracht und tiefgefroren wird ; und sämtliche Teilschichten gemeinsam lyophilisiert werden.

29. Verfahren nach Anspruch 28, dadurch gekennzeichnet, daß vor einem oder jedem Tieffrier-Vorgang verschäumt wird.

## Claims

1. A resorptive sheet material for closing and healing wounds, consisting essentially of a glycoprotein matrix containing substances which cause and, respectively, are conducive to the coagulation of blood including fibrinogen and thrombin, characterized in that the sheet material is dry and of multi-layered structure ; at least one layer (1, 4) is free from thrombin and contains in the glycoprotein matrix thereof, the fibrinogen in a substantially homogeneously distributed form ; and at least one further layer (2, 3) is free from fibrinogen and contains in the glycoprotein matrix thereof, the thrombin in a substantially homogeneously distributed form.

2. A sheet material according to claim 1, characterized in that at least one layer (1, 2, 3 and/or 4) contains fibroblast cells present in dry state.

3. A sheet material according to claim 1, characterized in that at least one layer (1, 2, 3 and/or 4) contains — apart from thrombin, where applicable — an agent which is specifically conducive to the growth spreading-in, or both of fibroblasts.

4. A sheet material according to claim 3, characterized in that the agent specifically conducive to the growth or spreading-in, or both of fibroblasts is chondroitin sulphate.

5. A sheet material according to anyone of claims 1 to 4, characterized in that the sheet material is double-layered.

6. A sheet material according to claim 5, characterized in that each layer (1, 2) of the double-layered sheet material (Fig. 1) has a foam or fleece structure ; and each layer (1, 2) has a layer thickness ranging from 1 to 5 mm.

7. A sheet material according to anyone of claims 1 to 4, characterized in that the sheet material is three-layered.

8. A sheet material according to claim 7, characterized in that the centrally disposed layer (4) of the three-layered sheet material (Fig. 2) is free from thrombin and contains the fibrinogen in a substantially homogeneously distributed form ; each outer layer (3, 5) is free from fibrinogen ; and at least one outer layer (3, 5) contains the thrombin in a substantially homogeneously distributed form.

9. A sheet material according to claims 7 or 8, characterized in that the fibroblast cells and/or the agent specifically conducive to the growth or spreading-in, or both of fibroblasts, here especially chondroitin sulphate, are present in at least one of the outer layers.

10. A sheet material according to claim 7 characterized in that the glycoprotein of the centrally disposed layer (4) differs from that of the outer layers (3, 5) in its chemical nature or, when the same glycoprotein is concerned, in its density, its layer thickness, its degree of polymerization and/or its stability under mechanical load.

11. A sheet material according to anyone of claims 7 to 10, characterized in that the centrally disposed layer (4) of the three-layered sheet material is of greater layer thickness than the outer layers (3, 5) thereof.

12. A sheet material according to anyone of claims 7 to 11, characterized in that all layers (3, 4, 5) of the three-layered sheet material have a foam or fleece structure ;

the centrally disposed layer (4) has a layer thickness ranging from 3 to 8 mm ; and the outer layers (3, 5) respectively have a layer thickness ranging from 1 to 3 mm.

13. A sheet material according to anyone of claims 1 to 12, characterized in that the thrombin-containing layer contains 10 to 2 000 units of thrombin per 1 $cm^3$ of the glycoprotein matrix.

14. A sheet material according to anyone of claims 1 to 13, characterized in that the thrombin-containing layer additionally contains substances having a vasoactive effect, especially adrenaline and/or ergotamine.

15. A sheet material according to anyone of claims 2 to 14, characterized in that the fibroblast-containing layer contains $10^3$ to $10^{10}$ fibroblast cells per 1 $cm^3$ of the glycoprotein matrix.

16. A sheet material according to claim 15, characterised in that the fibroblast-containing layer additionally contains 0,1 to 1 mg of chon-droitin sulphate per 1 $cm^3$ of the glycoprotein matrix.

17. A sheet material according to anyone of claims 1 to 16, characterized in that the fibrinogen-containing layer contains 0,1 to 30 mg of fibrinogen per 1 $cm^3$ of the glycoprotein matrix.

18. A sheet material according to anyone of claims 1 to 17, characterized in that the sheet material has a length of 3 to 12 cm, a width of 1 to 12 cm and a total layer thickness of 5 to 20 mm.

19. A sheet material according to anyone of claims 1 to 18, characterized in that the glycoprotein is non-linked fibrin, a fibrin fission product, collagen, globulin, myoglobulin, casein or albumin or a mixture of two or more of these components.

20. A sheet material according to anyone of claims 1 to 19, characterized in that the glycoprotein matrix is obtained by foaming and/or deep-freezing a substantially homogeneous predominantly aqueous glycoprotein solution and/or suspension and subsequent lyophilization.

21. A sheet material according to anyone of claims 1 to 20, characterized in that the glycoprotein matrix forms a fleece the fibers of which are obtained by spinning a homogeneous, predominantly aqueous glycoprotein solution.

22. A method for preparing a sheet material according to one or several of claims 1 to 21, characterized by adding fibrinogen to a thrombin-free, predominantly aqueous solution and/or suspension of the glycoprotein and processing said solution and/or suspension to form a sheet-material layer ; and applying onto the full surface of this layer at least one further layer which, in its turn, has been obtained from a further, fibrinogen-free and thrombin-containing solution and/or suspension of the same or of another glycoprotein.

23. A method according to claim 22, characterized by additionally enriching the thrombin-containing solution or suspension of the glycoprotein with active fibroblast cells.

24. A method as claimed according to claims 22 or 23, characterized by additionally enriching the thrombin-containing solution or suspension of the glycoprotein with an agent conducive to the growth or spreading-in, or both of fibroblasts.

25. A method according to claim 24, characterized by additionally adding chondroitin sulphate to the thrombin-containing solution or suspension of the glycoprotein.

26. A method according to anyone of claims 22 to 25, characterized in that for producing the sheet-material layer, the respective solution and/or suspension is applied onto an inert surface, where it is subjected to deep-freezing and, finally, to lyophilization.

27. A method according to anyone of claims 22 to 26, characterized by producing the layers independently of one another ; moistening at least one layer surface with water ; and placing a further layer of different composition onto the moistened layer surface.

28. A method according to anyone of claims 22 to 26, characterized by applying a solution and/or suspension of one composition onto an inert surface, where it is subjected to deep-freezing ; and applying onto the resultant ice-layer surface, when still in the deep-frozen state, a solution and/or suspension of different composition and subjecting it to deep-freezing ; and jointly subjecting all layers to lyophilization.

29. A method as claimed in claim 28, characterized by foaming before one or each of the deep-freezing operations.

**Revendications**

1. Matériau plat résorbable pour fermer et pour cicatriser des plaies, constitué essentiellement d'une matrice glycoprotéinique ayant une certaine teneur en substances provoquant ou favorisant la coagulation du sang, y compris du fibrinogène et de la thrombine, caractérisé en ce que, le matériau plat est sec et est constitué de plusieurs couches ; au moins une couche partielle (1, 4) est exempte de thrombine et contient le fibrinogène répartie d'une manière sensiblement homogène dans sa matrice glycoprotéinique ; et au moins une autre couche partielle (2, 3) est exempte de fibrinogène et contient la thrombine répartie de manière sensiblement homogène dans sa matrice glycoprotéinique.

2. Matériau plat suivant la revendication 1, caractérisé en ce que, au moins une couche partielle (1, 2, 3 et/ou 4) contient des cellules de fibroplaste se présentant à l'état sec.

3. Matériau plat suivant la revendication 1 ou 2, caractérisé en ce que, au moins une couche partielle (1, 2, 3 et/ou 4) contient — outre, le cas échéant, de la thrombine — un agent favorisant la croissance et/ou l'apparition de fibroplastes.

4. Matériau plat suivant la revendication 3, caractérisé en ce que, l'agent favorisant, de manière délibérée, la croissance et/ou l'apparition de fibroplastes, est du sulfate de chondroïtine.

5. Matériau plat suivant l'une des revendications 1 à 4, caractérisé en ce que, le matériau plat est constitué de deux couches.

6. Matériau plat suivant la revendication 5, caractérisé en ce que, chaque couche partielle (1, 2) du matériau en deux couches (figure 1) a une structure mousse ou fibreuse ; et chaque couche partielle (1, 2) a une épaisseur comprise entre 1 et 5 mm.

7. Matériau plat suivant l'une des revendications 1 à 4, caractérisé en ce que, le matériau plat est disposé en trois couches.

8. Matériau plat suivant la revendication 7, caractérisé en ce que, la couche partielle (4) disposée au milieu du matériau plat en trois couches (figure 2) est exempte de thrombine et contient le fibrinogène réparti d'une manière sensiblement homogène ;

chaque couche partielle extérieure (3, 5) est exempte de fibrinogène ; et au moins une couche partielle extérieure (3, 5) contient la thrombine répartie d'une manière sensiblement homogène.

9. Matériau plat suivant la revendication 7 ou 8, caractérisé en ce que, les cellules de fibroplaste et/ou l'agent favorisant délibérément la croissance et/ou l'apparition de fibroplastes, dans ce cas notamment le sulfate de chondroïtine, sont contenus dans l'une au moins des couches partielles extérieures.

10. Matériau plat suivant l'une des revendications 7 à 9, caractérisé en ce que, la glycoprotéine de la couche partielle (4) disposée au milieu se distingue de celles des couches partielles extérieures (3, 5) par sa nature chimique ou, dans le cas où il s'agit de la même glycoprotéine, par sa densité, par son épaisseur, par son degré de polymérisation et/ou par son aptitude à supporter des charges mécaniques.

11. Matériau plat suivant l'une des revendications 7 à 10, caractérisé en ce que, la couche partielle (4) disposée au milieu du matériau plat à trois couches a une plus grande épaisseur que celle des couches partielles extérieures (3, 5).

12. Matériau plat suivant l'une des revendications 7 à 11, caractérisé en ce que, toutes les couches partielles (3, 4, 5) du matériau plat à trois couches ont une texture en mousse ou fibreuse ;

la couche partielle (4) disposée au milieu a une épaisseur comprise entre 3 et 8 mm ; et les couches partielles extérieures (3, 5) ont chacune une épaisseur de 1 à 3 mm.

13. Matériau plat suivant l'une des revendications 1 à 12, caractérisé en ce que, la couche partielle contenant de la thrombine contient de 10 à 2 000 unités de thrombine par $cm^3$ de matrice glycoprotéinique.

14. Matériau plat suivant l'une des revendications 1 à 13, caractérisé en ce que, la couche partielle contenant de la thrombine contient, en plus, des substances agissant sur les vaisseaux sanguins, notamment de l'adrénaline et/ou de l'ergotamine.

15. Matériau plat suivant l'une des revendications 2 à 4, caractérisé en ce que, la couche partielle contenant des fibroplastes contient de $10^3$ à $10^{10}$ cellules de fibroplastes par $cm^3$ de matrice glycoprotéinique.

16. Matériau plat suivant la revendication 15, caractérisé en ce que, la couche partielle contenant des fibroplastes contient, en outre, de 0,1 à 1 mg de sulfate de chondroïtine par $cm^3$ de matrice glycoprotéinique.

17. Matériau plat suivant l'une des revendications 1 à 16, caractérisé en ce que, la couche partielle contenant du fibrinogène contient de 0,1 à 30 mg de fibrinogènes par $cm^3$ de matrice glycoprotéinique.

18. Matériau plat suivant l'une des revendications 1 à 17, caractérisé en ce que, le matériau plat a une longueur de 3 à 12 cm, une largeur de 1 à 12 cm et une épaisseur totale de 5 à 20 mm.

19. Matériau plat suivant l'une des revendications 1 à 18, caractérisé en ce que, la glycoprotéine est de la fibrine non réticulée, un produit de décomposition de la fibrine, du collagène, une globuline, de la myoglobuline, de la caséine ou de l'albumine, ou un mélange de deux ou plusieurs de ces constituants.

20. Matériau plat suivant l'une des revendications 1 à 19, caractérisé en ce que, la matrice glycoprotéinique est obtenue par moussage et/ou refroidissement à très basse température, d'une solution et/ou d'une suspension glycoprotéinique sensiblement homogène et essentiellement aqueuse, suivie d'une lyophilisation.

21. Matériau plat suivant l'une des revendications 1 à 20, caractérisé en ce que, la matrice glycoprotéinique forme un tissu non tissé, dont les fibres ont été obtenues par filage d'une solution homogène essentiellement aqueuse de glycoprotéine.

22. Procédé de fabrication d'un matériau plat suivant l'une ou plusieurs des revendications 1 à 21, caractérisé en ce qu'il consiste, à mélanger une solution et/ou une suspension de glycoprotéines exempte de thrombine et essentiellement aqueuse, à du fibrinogène, et à la transformer en une couche partielle de matériau plat ; et à déposer, sur toute la surface de cette couche partielle, au moins une autre couche partielle qui a été obtenue, pour sa part, à partir d'une autre solution et/ou suspension exempte de fibrinogènes et contenant de la thrombine, de ladite glycoprotéine ou une autre glycoprotéine.

23. Procédé suivant la revendication 22, caractérisé en ce qu'il consiste, à enrichir la suspension ou la solution glycoprotéinique contenant de la thrombine, par des cellules de fibroplastes actives.

24. Procédé suivant la revendication 22 ou 23, caractérisé en ce qu'il consiste, à enrichir la solution ou la suspension glycoprotéinique contenant de la thrombine, d'un agent favorisant la croissance et/ou l'apparition de fibroplastes.

25. Procédé suivant la revendication 24, caractérisé en ce qu'il consiste, à ajouter du sulfate de chondroïtine à la solution ou à la suspension glycoprotéinique contenant de la thrombine.

26. Procédé suivant l'une des revendications 22 à 25, caractérisé en ce qu'il consiste, à déposer, pour obtenir la couche partielle de matériau plat, la solution et/ou la suspension sur une surface inerte, à l'y congeler à très basse température et, ensuite, à la lyophiliser.

27. Procédé suivant l'une des revendications 22 à 26, caractérisé en ce qu'il consiste, à produire les couches partielles indépendamment l'une de l'autre ;

à humidifier par de l'eau l'une au moins des surfaces de couches partielles ; et à déposer une autre couche partielle de composition différente sur la surface de la couche partielle humidifiée.

28. Procédé suivant l'une des revendications 22 à 26, caractérisé en ce qu'il consiste, à déposer une solution et/ou une suspension ayant une certaine composition sur une surface inerte et à l'y congeler à très basse température ; et à déposer, sur la surface de couche de glace ainsi obtenue, se trouvant encore à l'état refroidi à très basse température, une solution et/ou une suspension ayant une autre composition, et à l'y congeler à très basse température ; et à lyophiliser ensemble toutes les couches partielles.

29. Procédé suivant la revendication 28, caractérisé en ce qu'il consiste, à effectuer un moussage avant chaque processus de congélation à température très basse.

Fig. 1

Fig. 2